Europäisches Patentamt

**European Patent Office**

**Office européen des brevets**

(11) Veröffentlichungsnummer: **0 285 679**
**A1**

# EUROPÄISCHE PATENTANMELDUNG

(21) Anmeldenummer: **87105034.0**

(22) Anmeldetag: **04.04.87**

(51) Int. Cl.⁴ **A61M 5/14**

(43) Veröffentlichungstag der Anmeldung:
**12.10.88 Patentblatt 88/41**

(84) Benannte Vertragsstaaten:
**AT BE CH DE ES FR GB GR IT LI LU NL SE**

(71) Anmelder: **B. Braun-SSC AG**
**Gerliswilstrasse 74**
**CH-6020 Emmenbrücke(CH)**

(72) Erfinder: **Mauerer, Erich**
**Druseltalstrasse 87**
**D-3500 Kassel(DE)**
Erfinder: **Mengel, Rainer**
**Lessingstrasse 5**
**D-3501 Niederstein 3(DE)**

(74) Vertreter: **von Kreisler, Alek, Dipl.-Chem. et al**
**Deichmannhaus am Hauptbahnhof**
**D-5000 Köln 1(DE)**

(54) **Druckinfusionsapparat.**

(57) Der Druckinfusionsapparat zum Ausdrücken einer Spritze (22) weist einen Absolut-Weggeber (28) auf, der mit dem bewegbaren Halter (25) verbunden ist und die Positionsdaten der Stellung des Spritzenstößels (24) an ein Steuergerät (31) liefert. Unter Verwendung der Positionsdaten wird der Motor (11) so geregelt, daß eine gewünschte Infusionsrate eingehalten wird. Die Infusionsrate kann sich während eines Infusionsvorgangs programmgemäß verändern. Kurz vor Erreichen der Leer-Position wird Voralarm gegeben, und bei Erreichen der Leer-Position wird der Antrieb abgeschaltet.

EP 0 285 679 A1

## Druckinfusionsapparat

Die Erfindung betrifft einen Druckinfusionsapparat nach dem Oberbegriff des Patentanspruchs 1.

Zur Durchführung von Infusionen, d.h. zur Zuführung von Flüssigkeit zu einem Patienten, werden Druckinfusionsapparate benutzt, die eine die Flüssigkeit enthaltende Spritze fortlaufend ausdrücken. Die aus der Spritze ausgedrückte Flüssigkeit wird über einen Schlauch dem Patienten zugeführt. Ein bekannter Druckinfusionsapparat, von dem der Oberbegriff des Patentanspruchs 1 ausgeht (DE-C-3 150 623) weist ein langgestrecktes rohrförmiges Gehäuse auf, an dem ein erster Halter zum Abstützen des Spritzenzylinders befestigt ist. Das Gehäuse enthält einen Spindelantrieb, und aus seinem einen Ende ragt ein entlang der Gehäuseachse verschiebbarer Schlitten heraus, an dem ein zweiter Halter zum Ansetzen an den Spritzenstößel angebracht ist. Durch Drehen einer im Gehäuse angeordneten Spindel wird der Schlitten relativ zum Gehäuse linear verschoben, wodurch die in den Druckinfusionsapparat eingesetzte Spritze ausgedrückt wird. Der Antrieb der Vorschubvorrichtung erfolgt über einen Elektromotor.

Ferner ist es bekannt, an Druckinfusionsapparaten Endschalter oder Voralarmschalter vorzusehen, die bei Erreichen einer bestimmten Vorschubstellung ansprechen, um Alarm zu erzeugen oder das Gerät abzuschalten, wenn die Spritze den Leerzustand ganz oder nahezu erreicht hat. Solche Endschalter stellen jedoch einen zusätzlichen Aufwand dar, und sie müssen zudem regelmäßig auf ordnungsgemäßes Funktionieren überprüft werden.

Die bekannten Druckinfusionsapparate erfordern eine hohe Präzision der Vorschubvorrichtung und des elektrischen Antriebs, weil der gesamte Arbeitsvorgang vorher eingestellt wird und anschließend ohne jegliche Überwachung selbständig abläuft. Es muß daher sichergestellt werden, daß eine einmal eingestellte Infusionsgeschwindigkeit im weiteren Verlauf exakt eingehalten wird. Außerdem dürfen durch Fertigungsungenauigkeiten und Exemplarstreuungen keine zu großen Abweichungen auftreten.

Der Erfindung liegt die Aufgabe zugrunde, einen Druckinfusionsapparat der im Oberbegriff des Patentanspruchs 1 angegebenen Art zu schaffen, bei dem der gesamte Infusionsvorgang überwacht und gesteuert wird und bei dem eine genaue Einhaltung der Infusionsbedingungen sichergestellt ist.

Die Lösung dieser Aufgabe erfolgt erfindungsgemäß mit den im kennzeichnenden Teil des Patentanspruchs 1 angegebenen Merkmalen.

Bei dem erfindungsgemäßen Druckinfusionsapparat überwacht ein Weggeber den gesamten Verschiebeweg der Vorschubvorrichtung bzw. des bewegbaren Halters, mit dem die Spritze ausgedrückt wird. Die Positionsdaten werden an das Steuergerät gemeldet, das die Positionssteuerung entweder nach einem vorgegebenen Programm oder in Abhängigkeit von externen Meßdaten durchführt. Auf diese Weise wird der Ausdrückzustand der Spritze in jedem Stadium überwacht und dem Steuergerät mitgeteilt. Das Steuergerät ermittelt, ob der jeweilige Positionswert der für diesen Zeitpunkt vorgesehenen Soll-Position entspricht, und kann erforderlichenfalls Korrekturen vornehmen, indem die Vorschubgeschwindigkeit vorübergehend erhöht oder erniedrigt wird. Es besteht daher die Möglichkeit, die Infusionsrate nach einem Programm zeitabhängig oder mengenabhängig zu verändern, wobei beispielsweise zunächst eine hohe Infusionsrate eingestellt wird, die dann kontinuierlich verringert wird. Andererseits kann der Vorschub auch in Abhängigkeit von externen Meßdaten gesteuert werden, die beispielsweise Aufschluß über die Wirkung des infundierten Medikaments liefern. Bei Verabreichen eines blutdrucksenkenden Mittels kann die Infusionsrate in Abhängigkeit vom augenblicklichen Blutdruck des Patienten variiert werden.

Ein besonderer Vorteil besteht darin, daß nicht nur bestimmte Infusionsraten vorgegeben werden können, sondern das die Einhaltung dieser Infusionsraten auch genau überwacht und an das Steuergerät gemeldet wird. Etwaige Abweichungen können daher unter Anwendung bekannter Regelkriterien ausgeglichen werden.

Vorzugsweise ist der Weggeber ein Absolut-Weggeber, der die Position eines den Spritzenstößel bewegenden zweiten Halters in bezug auf eine feste Referenzposition feststellt. Da jeweils die Absolut-Position des zweiten Halters bekannt ist und da ferner die Leer-Position bekannt ist, die der zweite Halter einnimmt, wenn die Spritze vollständig ausgedrückt ist, sind Endschalter für die Erzeugung von Voralarm oder für die Endabschaltung des Antriebs nicht erforderlich. Die Leer-Position kann für den jeweiligen Spritzentyp manuell in eine Eingabevorrichtung des Steuergeräts eingegeben werden. Ferner besteht die Möglichkeit, die Leer-Position einzugeben, indem die Spritze im leeren Zustand in den Druckinfusionsapparat eingesetzt wird. Auf Knopfdruck kann dann die betreffende Position des zweiten Halters in das Steuergerät eingespeichert werden. Danach kann die Spritze aufgezogen werden, indem der Kolben im Zylinder zurückgezogen wird. Die sich dabei ergebende Stellung des zweiten Halters gibt (unter Berücksichtigung des Spritzenquerschnitts) Aufschluß über das in die Spritze aufgezogene Flüssigkeitsvolumen.

Das Steuergerät kann ferner die Spritze auf Blockierungen des Infusionsvorgangs überwachen. Wenn beispielsweise der an die Spritze angesetzte Schlauch abgeknickt ist und der weitere Vorschub behindert wird, obwohl die Leer-Position noch nicht erreicht ist, dann kann dies dadurch festgestellt werden, daß der Spritzenvorschub geringer ist als ein vorgegebener Wert, so daß Alarm ausgelöst werden muß. Es ist also nicht erforderlich, Belastungsmeßvorrichtungen vorzusehen, um das Blockieren der Infusion erkennen zu können.

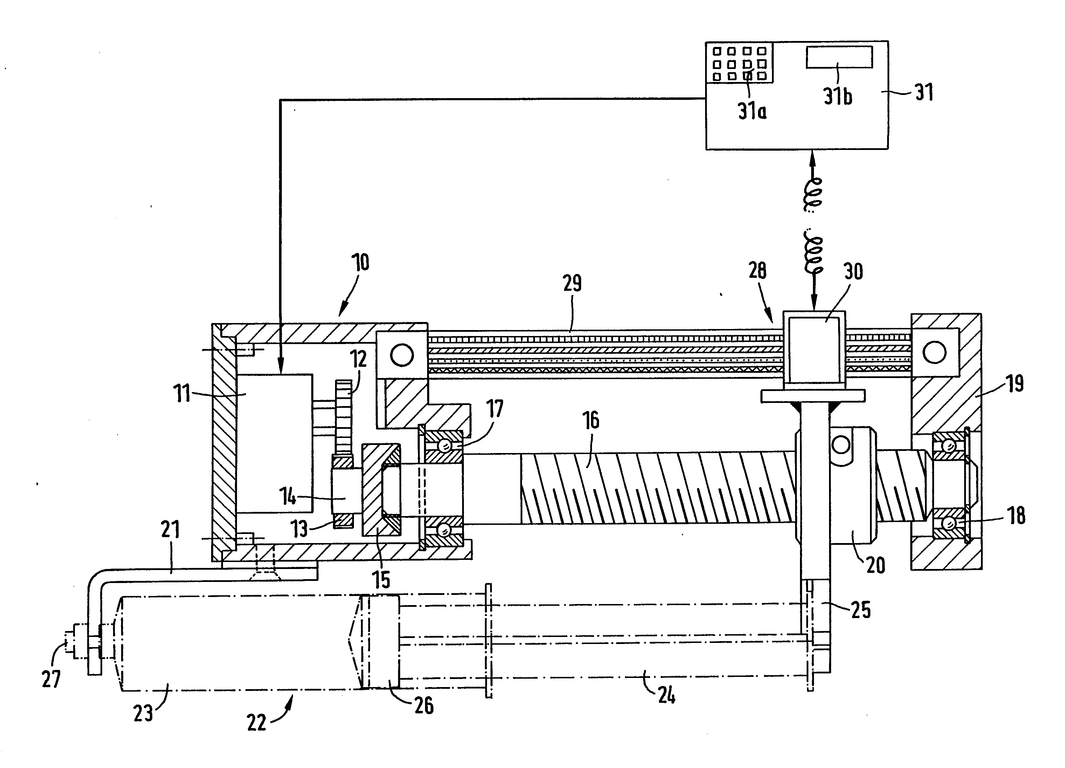

Im folgenden wird unter Bezugnahme auf die einzige Figur der Zeichnung ein Ausführungsbeispiel der Erfindung näher erläutert.

In der Zeichnung ist schematisch ein Längsschnitt durch einen Druckinfusionsapparat dargestellt.

Der Druckinfusionsapparat weist ein Gehäuse 10 auf, das eine Motor-Getriebe-Einheit 11 enthält, welche über Zahnräder 12,13 eine Welle 14 antreibt. Die Welle 14 ist über eine Kupplung 15 mit dem einen Ende der Gewindespindel 16 verbunden. In der Nähe dieses Endes ist die Gewindespindel 16 mit einem Lager 17 in der Wand des Gehäuses 10 gelagert. Die Gewindespindel 16 ragt aus dem Gehäuse 10 heraus, und ihr entgegengesetztes Ende ist mit einem Lager 18 in einem Block 19 gelagert, der über eine (nicht dargestellte) Brücke starr mit dem Gehäuse 10 verbunden ist. Auf der Gewindespindel 16 ist ein Schlitten 20 geführt, der als Spindelmutter ausgebildet ist und der mit seinem Innengewinde in Eingriff mit dem Außengewinde der Gewindespindel 16 steht. Der Schlitten 20 wird gegen Drehung festgehalten, so daß er sich bei Drehung der Gewindespindel 16 in Längsrichtung dieser Spindel bewegt. Die Teile 11 bis 20 bilden die Vorschubvorrichtung. Diese Vorschubvorrichtung kann alternativ auch so ausgebildet sein, wie diejenige des Druckinfusionsapparats nach DE-C-3 150 623.

An dem Gehäuse 10 ist der erste Halter 21 befestigt, in den der Hals des Spritzenzylinders 23 der Spritze 22 eingesetzt wird. Das Ende des Stößels 24 der Spritze wird in den zweiten Halter 25 eingesetzt, der fest mit dem Schlitten 20 verbunden ist. Die Spritze 22 enthält im Spritzenzylinder 23 einen mit dem Stößel 24 fest verbundenen Kolben 26, der die in der Spritze enthaltene Flüssigkeit aus dem Auslaß 27, an den ein Schlauch angeschlossen werden kann, austreibt.

Erfindungsgemäß ist ein Weggeber 28 vorgesehen, der die axiale Stellung des Schlittens 20 in bezug auf das Gehäuse 10 bzw. den ersten Halter 21 erkennt. Der Weggeber 28 weist eine mit Kodierungen versehene Schiene 29 auf, die sich zwischen dem Gehäuse 10 und dem Block 19 erstreckt, sowie einen längs der Schiene 29 bewegbaren Tastkopf 30, der mit dem zweiten Halter 25 fest verbunden ist. Der Tastkopf 30 ist mit mehreren Sensoren versehen, von denen jeder längs einer Signalspur der Signalschiene 29 bewegt wird. Die Signalschiene 29 weist magnetisch oder optisch abtastbare Signalspuren auf. Die Kombination der Signale der im Tastkopf 30 enthaltenen Sensoren gibt Auskunft über die Position des Tastkopfes 30 in Längsrichtung der Signalschiene 29. Im einzelnen entspricht der Positionsgeber 28 der Serie 500 der Firma MITUTOYO. Ein solcher Positionsgeber hat eine Auflösung von 0,01 mm.

Die Ausgangssignale des Tastkopfes 3, die die Positionssignale des zweiten Halters 25 darstellen, werden dem Steuergerät 31 zugeführt. Dieses Steuergerät enthält einen Mikroprozessor, der in Abhängigkeit von den Positionssignalen den Motor der Motor-Getriebe-Einheit 11 steuert. An einer Tastatur 31a des Steuergeräts 31 können verschiedene Parameter des Infusionsvorgangs in den Mikroprozessor eingegeben werden, beispielsweise der Innenquerschnitt des Spritzenzylinders 23, das in der Spritze befindliche Flüssigkeitsvolumen und die Leer-Position der Spritze, d.h. diejenige Position, die der Halter 25 einnimmt, wenn die Spritze vollständig ausgedrückt ist. Ferner kann die gewünschte Infusionsrate, nämlich das auszudrückende Flüssigkeitsvolumen, pro Zeiteinheit eingegeben werden. Es ist auch möglich, mehrere verschiedene Infusionsraten, die nacheinander bei demselben Infusionsvorgang wirksam werden sollen, einzugeben. Der Mikroprozessor berechnet aus den eingegebenen Daten die jeweilige Infusionsgeschwindigkeit. Anhand einer Zeitmessung ermittelt er, ob die jeweilige Ist-Infusionsrate der Soll-Infusionsrate entspricht. In Abhängigkeit von der sich ergebenden Abweichung wird die Drehzahl des Motors korrigiert.

Das Steuergerät 31 kann die Regelung entweder anhand der ihr eingegebenen und der ihr von dem Tastkopf 28 zugeführten Wegdaten vornehmen oder anhand der Volumendaten. Die Volumendaten sind das Produkt aus Weg-und Querschnittsfläche des Spritzenzylinders 23.

An der Anzeigevorrichtung 31b des Steuergerätes 31 kann das schon aus der Spritze ausgedrückte Flüssigkeitsvolumen oder das noch in der Spritze enthaltene Flüssigkeitsvolumen angezeigt werden. Außerdem kann die noch verfügbare Infusionsdauer angezeigt werden.

Die Leer-Position, die der Tastkopf 30 bzw. der zweite Halter 25 einnehmen, wenn die Spritze 22 vollständig ausgedrückt ist, kann entweder an der Tastatur 31a manuell eingegeben werden oder sie kann dadurch eingegeben werden, daß eine leere Spritze, die keine Flüssigkeit enthält, mit vorgeschobenem Kolben in die Halter 21 und 25 eingesetzt wird. Die Position, die der Halter 25 in diesem Zustand einnimmt, wird durch Druck einer Taste am Steuergerät 31 in dieses eingespeichert. Der Druckinfusionsapparat kann dann anschließend dazu benutzt werden, Flüssigkeit in die Spritze 22 einzuziehen, indem der Schlitten 20 den Stößel 24 zurückzieht. Bei Beendigung des Aufziehens wird der Positionswert des Tastkopfes 30 im Steuergerät 31 ebenfalls gespeichert. Auf diese Weise sind im Steuergerät Informationen über das Volumen der in die Spritze aufgezogenen Flüssigkeitsmenge enthalten.

Andererseits ist es möglich, eine Spritze, deren Abmessungen bekannt und im Steuergerät 31 gespeichert sind, zu verwenden und unter kontrollierter Steuerung auszudrücken.

Bei Erreichen einer Position, die um ein bestimmtes Maß vor der Leer-Position der Spritze liegt, erzeugt das Steuergerät 31 Alarm, damit das Personal weiß, daß der Infusionsvorgang in Kürze beendet sein wird und daß ggf. eine neue Spritze einzusetzen ist. Die Position, bei der der Voralarm erzeugt wird, kann auch so gewählt werden, daß sich eine bestimmte Restinfusionszeit ergibt. In diesem Fall variiert die Position, bei der Voralarm erzeugt wird, in Abhängigkeit von der Vorschubgeschwindigkeit.

Das Steuergerät 31 kontrolliert ferner, ob die Vorschubgeschwindigkeit oder die Infusionsrate einen vorgegebenen Mindestwert nicht unterschreitet. Wird dieser Mindestwert unterschritten, weil beispielsweise der an die Spritze 22 angeschlossene Schlauch abgeknickt oder anderweitig verschlossen ist, dann erkennt das Steuergerät die Unterschreitung der Mindestgeschwindigkeit und erzeugt Alarm.

Ein einziges Steuergerät kann in Verbindung mit mehreren Druckinfusionsapparaten betrieben werden, von denen jeder einen Weggeber 28 aufweist. Die Druckinfusionsapparate lassen sich auf diese Weise miteinander koordinieren, was beispielsweise dann zweckmäßig ist, wenn einem Patienten mehrere Infusionen gleichzeitig in einem bestimmten Mengenverhältnis zugeführt werden sollen. Wenn ein Druckinfusionsapparat nicht ordnungsgemäß arbeitet, können beispielsweise alle Druckinfusionsapparate abgeschaltet werden. Ferner besteht die Möglichkeit, sämtliche Druckinfusionsapparate so zu betreiben, daß die Infusionslösungen dem Patienten in einem bestimmten vorgegebenen Mengenverhältnis, das exakt eingehalten werden kann, zugeführt werden. Ferner kann einer der Druckinfusionsapparate als Master benutzt werden, während die übrigen Druckinfusionsapparate entsprechend den vom Weggeber des Mastergerätes erzeugten Wegsignalen nachgeführt werden.

Das erfindungsgemäße Druckinfusionsgerät erlaubt es, selbst kleinste Flüssigkeitsmengen aus einem beliebig großen Volumen mit hoher Genauigkeit abzumessen und zu applizieren. Die an die Vorschubvorrichtung und den Antrieb zu stellenden Genauigkeitsanforderungen sind sehr gering, da die hohe Systemgenauigkeit durch den Weggeber erreicht wird.

## Ansprüche

1. Druckinfusionsapparat zum Ausdrücken einer Spritze (22), mit einem Halter (21) zum Abstützen der Spritze (22) und einer Vorschubvorrichtung (11-20) zum Ausdrücken der Spritze,
**dadurch gekennzeichnet,**
daß ein Weggeber (28) vorgesehen ist, der die Vorschubpositionen über den gesamten Verschiebeweg der Vorschubvorrichtung ermittelt und an ein Steuergerät (31) liefert, welches die Vorschubbewegung nach einem vorgespeicherten Programm oder in Abhängigkeit von externen Meßdaten steuert.

2. Druckinfusionsapparat nach Anspruch 1, dadurch gekennzeichnet, daß der Weggeber (28) ein Absolut-Weggeber ist, der die Positionen eines den Spritzenstößel (24) bewegenden zweiten Halters (25) in bezug auf eine feste Referenzposition feststellt.

3. Druckinfusionsapparat nach Anspruch 1 oder 2, dadurch gekennzeichnet, daß in das Steuergerät (31) eine Leer-Position eingebbar ist, welche der Weggeber (28) angibt, wenn die Spritze (22) ausgedrückt ist.

4. Druckinfusionsapparat nach Anspruch 3, dadurch gekennzeichnet, daß die Leer-Position in das Steuergerät (31) eingebbar ist, indem derjenige Positionswert, den der Weggeber (28) bei eingesetzter ausgedrückter Spritze (22) angibt, auf Knopfdruck gespeichert wird.

5. Druckinfusionsapparat nach Anspruch 3 oder 4, dadurch gekennzeichnet, daß das Steuergerät (31) die Differenz zwischen Ist-Position und Leer-Position berechnet und Voralarm erzeugt, wenn diese Differenz einen Grenzwert unterschreitet.

6. Druckinfusionsapparat nach einem der Ansprüche 1 bis 5, dadurch gekennzeichnet, daß das Steuergerät (31) aufgrund eingegebener Kenndaten der verwendeten Spritze (22) den Vorschubweg in das Flüssigkeitsvolumen umrechnet.

7. Druckinfusionsapparat nach Anspruch 6, dadurch gekennzeichnet, daß am Steuergerät (31) eine Anzeigevorrichtung (31b) vorgesehen ist, an der das schon ausgedrückte Flüssigkeitsvolumen oder das noch in der Spritze (22) enthaltene Flüssigkeitsvolumen anzeigbar ist.

8. Druckinfusionsapparat nach einem der Ansprüche 1 bis 7, dadurch gekennzeichnet, daß das Steuergerät (31) ermittelt, ob jeweils in einem vorgegebenen Zeitintervall ein bestimmter Mindestvorschub durchgeführt wurde, und Alarm erzeugt, wenn der Mindestvorschub nicht erreicht wurde.

9. Druckinfusionsapparat nach einem der Ansprüche 1 bis 8, dadurch gekennzeichnet, daß ein Steuerwerk (31) mehrere Vorschubvorrichtungen für jeweils eine Spritze (22) gleichzeitig steuert, wobei jede Vorschubvorrichtung einen eigenen Weggeber (28) aufweist.

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl.3) |
|---|---|---|---|
| Y | EP-A-0 171 337 (HOSPAL AG)<br>* Ansprüche 1-9; Figur 1 *<br>--- | 1-3,8 | A 61 M 5/14 |
| Y | WO-A-8 502 546 (DISETRONIC)<br>* Anspruch 1; Figur 1 *<br>--- | 1-3,8 | |
| A | FR-A-2 546 068 (SOCIETE DE FOURNITURES D'EQUIPEMENTS MEDICAUX)<br>* Ansprüche 1, 3 *<br>--- | 6 | |
| A | US-A-4 255 096 (COKER)<br>* Anspruch 7 *<br>----- | 9 | |

RECHERCHIERTE SACHGEBIETE (Int. Cl.3)

A 61 M 5/00

Der vorliegende Recherchenbericht wurde für alle Patentansprüche erstellt

| Recherchenort | Abschlußdatum der Recherche | Prüfer |
|---|---|---|
| BERLIN | 13-11-1987 | PAPA E.R. |